# EUROPEAN PATENT APPLICATION

(11) **EP 4 825 867 A1**
(43) Date of publication of application: **21.10.2026**
(21) Application number: 24904422.3
(22) Date of filing: 13.12.2024
(51) Int. Cl.: C07K 14/005, A61K 39/155, A61P 31/14, A61K 39/12, C07K 16/10

(54) **MUTANT RSV PROTEIN**

(30) Priority: 15.12.2023 KR 20230183501; 16.07.2024 KR 20240093898
(71) Applicant: SK Bioscience Co., Ltd., Gyeonggi-do 13494 (KR)
(72) Inventor: HONG, Seung-Hye, Seongnam-si, Gyeonggi-do 13494 (KR); KIM, Ju-hyung, Seongnam-si, Gyeonggi-do 13494 (KR); SEO, Ki-Weon, Seongnam-si, Gyeonggi-do 13494 (KR); HAN, Young-woo, Seongnam-si, Gyeonggi-do 13494 (KR)
(74) Representative: Meissner Bolte Partnerschaft mbB
(86) International application number: PCT/KR2024/096904
(87) International publication number: WO 2025/127857

(57) **Abstract**

The present invention relates to a recombinant Respiratory Syncytial Virus (RSV) F protein, an isolated nucleic acid molecule encoding the recombinant RSV F protein, a vector carrying the nucleic acid molecule, and a host cell. The invention also relates to a method for producing the recombinant RSV F protein. Furthermore, the invention relates to an immunogenic composition comprising the recombinant RSV F protein or the nucleic acid molecule encoding the recombinant RSV F protein.

## Description

### [Technical Field]

The present invention relates to a recombinant RSV (Respiratory syncytial virus) F protein, an isolated nucleic acid molecule encoding the recombinant RSV F protein, a vector comprising the nucleic acid molecule, and a host cell comprising the nucleic acid molecule. In addition, the present invention relates to a method for producing a recombinant RSV F protein. In addition, the present invention relates to an immunogenic composition comprising a recombinant RSV F protein or a nucleic acid molecule encoding the recombinant RSV F protein.

### [Background Art]

Respiratory syncytial virus (RSV) is a negative-sense single-strand non-segmented RNA virus belonging to the family Paramyxoviridae. RSV causes lower and upper respiratory tract infections in humans, and although infants and young children are the primary targets of infection, it may also cause fatal respiratory disease in immunocompromised patients and the elderly.

Currently, Nirsevimab and Palivizumab are used as prophylactic monoclonal antibody injections administered to infants and young children to reduce the risk of severe RSV, and Arexvy, developed by GSK, as an RSV subunit vaccine for elderly vaccination, Abrysvo, developed by Pfizer, as an RSV subunit vaccine for elderly individuals and pregnant women vaccination, and mRESVIA, developed by Moderna, as an RSV mRNA vaccine for elderly vaccination, have each been approved by the US Food and Drug Administration (FDA). The above two antibody injections target the F protein of RSV, and the above three vaccines contain the stabilized prefusion recombinant F protein of RSV or mRNA thereof as an antigen.

The F protein of RSV is a fusion glycoprotein abundant in the envelope and exists on the viral surface in a trimeric "prefusion" state. Once entry is triggered, it inserts a hydrophobic fusion loop into the cell membrane and undergoes extensive conformational changes to fold into an energetically favorable trimeric "post-fusion" state, fusing the viral and cell membranes together in this process.

The F protein of RSV is initially expressed as a single 574-residue polypeptide precursor ("F0" or "F0 precursor") containing a signal peptide sequence (residues 1-25) at the N-terminus and an ectodomain (ED) including three heptad repeats (HRA, HRB, and HRC), a transmembrane domain (TM), and a cytoplasmic tail (CT). Upon translation, the signal peptide is removed by a signal peptidase in the endoplasmic reticulum, and the remaining portion of the F0 precursor (i.e., residues 26-574) is further cleaved by a cellular protease (furin) at two multibasic sites (residues 109/110 and 136/137) to remove a 27-amino acid intervening sequence (residues 110-136) designated as p27, and to form the RSV F protomer, which is a heterodimer in which F1 (residues 137-574) and F2 (residues 26-109) are linked together via two disulfide bonds. Three protomers are assembled to form the final RSV F protein complex (a homotrimer of the three protomers).

The RSV F protein stabilized in a prefusion form is known to generate a greater neutralizing immune response in an animal model than that observed with the post-fusion form of the RSV F protein. For example, "DS-Cav1," which comprises S155C, S290C, S190F, and V207L substitutions, is a recombinant RSV F protein stabilized in a prefusion form, and has been reported to induce a neutralizing immune response several times greater than the immune response observed with the post-fusion form of the RSV F protein (McLellan et al., Science (2013) vol. 342, p. 592-598; International Patent Publication WO2014160463). In addition, "DS2" variants, which are second-generation antigens with improved structural stability and immunoprotective response compared to "DS-Cav1," have genetically linked F subunits, a fusion peptide deletion, and interprotomer movement stabilized by additional disulfide bonds and specific mutations, and have been reported to improve the antigen stability and immunogenicity of DS-Cav1 (Joyce et al., Nat Struct Mol Biol. (2016) 23(9): 811-820).

### [Brief Summary of Invention]

### [Technical Problem]

Accordingly, there is a need to develop a vaccine that achieves strong protection against RSV by further improving the stability and immunogenicity of RSV F proteins stabilized in a prefusion form, such as DS-Cav1 or DS2 variants.

### [Technical Solution]

An example of the present disclosure provides a recombinant RSV (Respiratory syncytial virus) F protein comprising an F2 polypeptide of positions 26-109 and an F1 polypeptide of positions 137-513 based on the sequence of a wild-type RSV F protein precursor polypeptide of SEQ ID NO: 1, and comprising S155C, S290C, S190F, V207L, D486L, E487L, and F488W amino acid substitutions.

Another example of the present disclosure provides a recombinant RSV F protein comprising an F2 polypeptide at positions 26-103 and an F1 polypeptide at positions 145-513 based on the sequence of a wild-type RSV F protein precursor polypeptide of SEQ ID NO: 1, wherein a Glycine(Gly)-Serine(Ser) peptide linker is introduced between the amino acid residues at positions 103 and 145, and wherein the recombinant RSV F protein further comprises S155C, S290C, S190F, V207L, L373R, D486L, E487L, and F488W amino acid substitutions.

Another example of the present disclosure provides a recombinant RSV F protein, further comprising the following amino acid substitutions: (a) A149C and Y458C; (b) N183GC and N428C; or (c) A149C, Y458C, S46G, E92D, S215P, and K465Q.

Another example of the present disclosure provides an isolated nucleic acid molecule encoding the recombinant RSV F protein.

Another example of the present disclosure provides a vector comprising the nucleic acid molecule.

Another example of the present disclosure provides an isolated host cell comprising the nucleic acid molecule.

Another example of the present disclosure provides a method for producing a recombinant RSV F protein from an isolated host cell comprising the nucleic acid molecule.

Another example of the present disclosure provides an immunogenic composition comprising the recombinant RSV F protein or a nucleic acid molecule encoding the recombinant RSV F protein.

### [Brief Description of Drawings]

FIG. 1 shows the results of an in-silico analysis to evaluate changes in the structural stability and interactions of the RSV Fusion protein in strains (A2 and B18537 strains) containing the DS-Cav1 prefusion F protein or the DS-Cav1_SK prefusion F protein, in which substitutions from DEF to LLW are additionally introduced at amino acid residues 486-488.
FIG. 2 shows the results of an *in vitro* thermostability test on strains (A2 and B18537 strains) containing the DS-Cav1 prefusion F protein or the DS-Cav1_SK prefusion F protein, in which substitutions from DEF to LLW is additionally introduced at amino acid residues 486-488.
FIG. 3 shows the results of measuring RSV neutralizing antibody titers in serum when strains (A2 and B18537 strains) containing the DS-Cav1 prefusion F protein or the DS-Cav1_SK prefusion F protein, in which substitutions from DEF to LLW are additionally introduced at amino acid residues 486-488, are administered to an animal model.
FIG. 4 shows the results of measuring serum prefusion F-specific IgG titers and RSV neutralizing antibody titers when the animal model is administered with strains (A2 and B18537 strains) comprising the WT, DS-Cav1, DS2-1, DS2-2, DS2-3, or DS2-4 prefusion RSV F protein, respectively.
FIG. 5 shows the results of measuring serum prefusion F-specific IgG titers and RSV neutralizing antibody titers when strains (A2 and B18537 strains) comprising a DS-Cav1, DS2-1, DS2-2, DS2-3, or DS2-4 prefusion RSV F protein, and strains (A2 and B18537 strains) comprising a DS-Cav1_SK, DS2-1 SK, DS2-2 SK, DS2-3 SK, or DS2-4 SK prefusion RSV F protein, in which substitutions from DEF to LLW are additionally introduced at amino acid residues 486-488, are each administered to an animal model.
FIG. 6 shows the results of measuring RSV neutralizing antibody titers in serum when strains (A2 and B18537 strains) comprising a DS-Cav1, DS2-1, DS2-2, DS2-3, or DS2-4 prefusion RSV F protein are administered to an animal model alone or in combination at a dose of 0.2, 1, or 5 µg.
FIG. 7 shows the results of measuring serum prefusion F-specific IgG titers and RSV neutralizing antibody titers when various prefusion RSV F proteins comprising or not comprising substitutions from DEF to LLW at amino acid residues 486-488 are administered as mRNA vaccines to an animal model.

### [Best Mode for Carrying Out the Invention]

According to one aspect of the present invention, there is provided a recombinant RSV (Respiratory syncytial virus) F protein comprising an F2 polypeptide of positions 26-109 and an F1 polypeptide of positions 137-513 based on the sequence of a wild-type RSV F protein precursor polypeptide of SEQ ID NO: 1, and comprising S155C, S290C, S190F, V207L, D486L, E487L, and F488W amino acid substitutions.

In one embodiment, the recombinant RSV F protein may comprise an F2 polypeptide of positions 26-103 and an F1 polypeptide of positions 145-513 based on the sequence of a wild-type RSV F protein precursor polypeptide of SEQ ID NO: 1, wherein a Glycine(Gly)-Serine(Ser) peptide linker is introduced between amino acid residues at positions 103 and 145, and the protein may further comprise S155C, S290C, S190F, V207L, L373R, D486L, E487L, and F488W amino acid substitutions.

In one embodiment, the recombinant RSV F protein may further comprise the following amino acid substitutions: (a) A149C and Y458C; (b) N183GC and N428C; or (c) A149C, Y458C, S46G, E92D, S215P, and K465Q.

In one embodiment, the recombinant RSV F protein may have a trimerization domain linked to the C-terminus directly or via a linker. For example, the trimerization domain may be a foldon domain.

In one embodiment, the recombinant RSV F protein may be derived from the F protein of RSV subtype A, the F protein of RSV subtype B, or the bovine RSV F protein.

In one embodiment, the recombinant RSV F protein may comprise the F2 polypeptide and the F1 polypeptide linked directly or via a linker.

In one embodiment, the recombinant RSV F protein may be soluble.

In one embodiment, the recombinant RSV F protein may exist as a trimer.

According to another aspect of the present invention, there is provided an isolated nucleic acid molecule encoding the recombinant RSV F protein.

In one embodiment, the nucleic acid molecule may encode a precursor polypeptide of a recombinant RSV F protein.

In one embodiment, the precursor polypeptide may comprise, in order from the N-terminus to the C-terminus, a signal peptide, an F2 polypeptide, a p27 polypeptide, an extracellular domain of F1 polypeptide, and a trimerization domain.

In one embodiment, the nucleic acid molecule may be an RNA molecule.

According to another aspect of the present invention, there is provided a vector comprising the nucleic acid molecule.

According to another aspect of the present invention, there is provided an isolated host cell comprising the nucleic acid molecule.

According to another aspect of the present invention, there is provided a method for producing a recombinant RSV F protein, which comprises introducing the nucleic acid molecule into an isolated host cell; and obtaining the recombinant RSV F protein by culturing the host cell.

According to another aspect of the present invention, there is provided an immunogenic composition comprising the recombinant RSV F protein or a nucleic acid molecule encoding the recombinant RSV F protein.

In one embodiment, the nucleic acid molecule may encode a recombinant precursor polypeptide of the RSV F protein.

In one embodiment, the precursor polypeptide may comprise, in order from the N-terminus to the C-terminus, a signal peptide, an F2 polypeptide, a p27 polypeptide, an extracellular domain of F1 polypeptide, and a trimerization domain.

In one embodiment, the nucleic acid molecule may be an RNA molecule.

In one embodiment, the immunogenic composition may be a vaccine composition.

Hereinafter, the present invention will be described in more detail.

When the terms "comprise," "comprises," "comprised," or "comprising" are used in this specification (including the claims), they are to be interpreted as specifying the presence of the stated features, integers, steps, or components, but not as excluding the presence of one or more other features, integers, steps, components, or groups thereof.

Descriptions of documents, statutes, materials, devices, articles, and the like in this specification are included solely for the purpose of providing context for the present invention. It is not suggested or represented that all or part of these form part of the prior art base or were common general knowledge in the field to which the present invention pertains before the priority date of each claim of this application.

Unless defined otherwise, all technical and scientific terms used herein have the same meaning as commonly understood by one of ordinary skill in the art to which the present invention belongs. It should also be understood that the terms and related definitions used herein are used for descriptive purposes only and are not intended to be limiting.

As used herein, the term "precursor polypeptide of RSV F protein" refers to a single 574-amino acid polypeptide initially present to express the RSV F protein in cells, and is also referred to as an "F0 polypeptide" or "F0 precursor." A wild-type precursor polypeptide typically comprises, in order from the N-terminus to the C-terminus, a signal peptide (residues 1-25), an F2 polypeptide (residues 26-109), a p27 polypeptide (residues 110-136), and an F1 polypeptide (residues 137-574). Upon translation, the signal peptide is removed by a signal peptidase in the endoplasmic reticulum, and the remaining portion of the F0 precursor (i.e., residues 26-574) is further cleaved by a cellular protease (furin) at two multibasic sites (residues 109/110 and 136/137) to remove the p27 polypeptide (residues 110-136) and form the RSV F protomer, which is a heterodimer in which the F1 and F2 polypeptides are linked together via two disulfide bonds. Three protomers are assembled to form the RSV F protein complex (a homotrimer of the three protomers).

The term "F1 polypeptide (F1)" refers to a polypeptide corresponding to residues 137-574 in the F0 precursor, and the wild-type F1 polypeptide consists of, in order from the N-terminus to the C-terminus, an extracellular domain (approximately residues 137-524), a transmembrane domain (approximately residues 525-550), and a cytoplasmic domain (approximately residues 551-574). In addition, the F1 polypeptide comprises a hydrophobic fusion peptide (fusion peptide) at the N-terminus and also comprises two heptad-repeat regions (HRA and HRB), where HRA is in the vicinity of the fusion peptide and HRB is in the vicinity of the transmembrane domain.

The term "F2 polypeptide (F2)" refers to a polypeptide corresponding to residues 26-109 in the F0 precursor.

The term "soluble F protein" refers to an F protein in a soluble form for use as an RSV vaccine. For example, the soluble F protein may be an F protein in which some or all of the transmembrane domain and cytoplasmic domain are deleted.

As used herein, the recombinant RSV F protein may be soluble, and thus the transmembrane domain and cytoplasmic domain of the wild-type RSV F protein may be deleted. For example, residues 514 to 574 may be deleted from the sequence of the wild-type precursor polypeptide of the RSV F protein.

In a preferred embodiment, the recombinant RSV F protein of the present disclosure may refer to a soluble F protein stabilized in a prefusion form. For example, it may refer to a protein comprising at least one epitope specific for the prefusion form of the RSV F protein, which is determined by specific binding of an antibody specific for the prefusion form and may be produced (expressed) in a sufficient amount. An "antibody specific for the prefusion form" refers to an antibody that specifically binds to the RSV F protein in the prefusion form but does not bind to the RSV F protein in the post-fusion form, and examples thereof contain D25, AM22, 5C4, MPE8, and AM14 antibodies.

For the purpose of use as a vaccine, the present invention is characterized by providing a recombinant RSV F protein comprising various modifications for stabilization in a prefusion form or for increasing antigen stability and/or immunogenicity.

An example of the present disclosure provides a recombinant RSV (Respiratory syncytial virus) F protein comprising an F2 polypeptide of positions 26-109 and an F1 polypeptide of positions 137-513 based on the sequence of a wild-type RSV F protein precursor polypeptide of SEQ ID NO: 1, and comprising S155C, S290C, S190F, V207L, D486L, E487L, and F488W amino acid substitutions.

Specifically, the substitution of Ser residues with Cys residues at residues 155 and 290 (S155C and S290C) prevents flipping to the post-fusion state by introducing a disulfide bond, and the cavity-filling mutations S190F and V207L may strengthen the F protein structure and stabilize its structure by introducing amino acids with larger volumes. Such an RSV F protein comprising the S155C, S290C, S190F, and V207L substitutions may generate neutralizing antibodies much more strongly than the post-fusion F protein.

In addition, the D486L, E487L, and F488W substitutions structurally stabilize the F protein in the prefusion form, and when the D486L, E487L, and F488W substitutions are additionally contained compared to the case where only the S155C, S290C, S190F, and V207L substitutions are contained, the structural stability and thermostability of the F protein in the prefusion form may be increased to increase the neutralizing antibody titer.

The present disclosure also provides a recombinant RSV F protein comprising an F2 polypeptide of positions 26-103 and an F1 polypeptide of positions 145-513 based on the sequence of a wild-type RSV F protein precursor polypeptide of SEQ ID NO: 1, wherein a Glycine (Gly)-Serine (Ser) peptide linker is introduced between the amino acid residues at positions 103 and 145, and the protein further comprises S155C, S290C, S190F, V207L, L373R, D486L, E487L, and F488W amino acid substitutions.

Specifically, the structural stability may be improved and the neutralizing antibody titer may be increased by additionally deleting some of the amino acid residues from the F1 polypeptide and the F2 polypeptide (e.g., additionally deleting 104-109 from the F2 polypeptide and deleting 137-144 corresponding to the fusion peptide from the F1 polypeptide) and introducing a GS linker between positions 105 and 145.

The present disclosure also provides a recombinant RSV F protein, further comprising the following amino acid substitutions: (a) A149C and Y458C; (b) N183GC and N428C; or (c) A149C, Y458C, S46G, E92D, S215P, and K465Q.

Specifically, the A149C and Y458C substitutions and the N183GC and N428C substitutions each introduce an interprotomer disulfide bond to increase interprotomer stability, thereby further increasing the physical stability and immunogenicity of the protein. In addition, the L373R, S46G, E92D, S215P, and K465Q mutations may increase the expression level of the interprotomer variant, increase the physical stability of the prefusion form, and further increase the immunogenicity.

As used herein, the numbering of amino acid residue positions is based on the sequence of the wild-type precursor polypeptide of the RSV F protein, for example, the amino acid sequence of the A2 strain shown in SEQ ID NO: 1. The sequence of SEQ ID NO: 1 comprises three naturally occurring substitutions (P102A, I379V, and M447V) in the F0 amino acid sequence of the RSV A2 strain (UniProtKB/Swiss-Prot: P03420).

**[Table 1]**

| Precursor | Sequence | SEQ ID NO |
|---|---|---|
| wild-type F0 | MELLILKANAITTILTAVTFCFASGQNITEEFYQSTCSAV SKGYLSALRTGWYTSVITIELSNIKENKCNGTDAKVKLI KQELDKYKNAVTELQLLMQSTPATNNRARRELPRFMN YTLNNAKKTNVTLSKKRKRRFLGFLLGVGSAIASGVA VSKVLHLEGEVNKIKSALLSTNKAVVSLSNGVSVLTSK VLDLKNYIDKQLLPIVNKQSCSISNIETVIEFQQKNNRLL EITREFSVNAGVTTPVSTYMLTNSELLSLINDMPITNDQ KKLMSNNVQIVRQQSYSIMSIIKEEVLAYVVQLPLYGVI DTPCWKLHTSPLCTTNTKEGSNICLTRTDRGWYCDNA GSVSFFPQAETCKVQSNRVFCDTMNSLTLPSEVNLCNV DIFNPKYDCKIMTSKTDVSSSVITSLGAIVSCYGKTKCT ASNKNRGIIKTFSNGCDYVSNKGVDTVSVGNTLYYVN KQEGKSLYVKGEPIINFYDPLVFPSDEFDASISQVNEKIN QSLAFIRKSDELLHNVNAGKSTTNIMITTIIIVIIVILLSLI AVGLLLYCKARSTPVTLSKDQLSGINNIAFSN | 1 |

The wild-type RSV F protein exhibits significant sequence conservation across RSV subtypes. For example, across the F0 precursor molecule, F proteins of subtypes A and B have approximately 90% amino acid sequence identity, and RSV subtypes A and B each have 81% sequence identity with bovine RSV F protein. Within RSV subtypes, the F0 sequence identity is much greater, for example, in each of the RSV A, B, and bovine subtypes, the RSV F0 precursor protein has approximately 98% sequence identity. Almost all identified RSV F0 precursor sequences are 574 amino acids long, typically differing slightly in length due to the length of the C-terminal cytoplasmic tail. In light of such significant sequence conservation, one of ordinary skill in the art may easily identify RSV F amino acid positions corresponding to the reference sequence of SEQ ID NO: 1 even in different RSV strains. For example, the amino acid positions presented herein may be numbered with reference to the amino acid numbering of SEQ ID NO: 1 by aligning sequences of other RSV strains with the sequence of SEQ ID NO: 1. Therefore, it should be noted that different RSV F0 sequences may have different numbering systems if, for example, there are amino acid residues added or removed compared to SEQ ID NO: 1.

In another embodiment, the recombinant RSV F protein of the present disclosure may have a trimerization domain linked to the C-terminus directly or via a linker.

The term "trimerization domain" refers to a domain that may promote trimer formation of an F1-F2 heterodimer. A number of exogenous multimerization domains that promote stable trimer formation of soluble proteins are known in the art. For example, the trimerization domain may be a foldon domain derived from bacteriophage T4 fibritin, comprising the amino acid sequence GYIPEAPRDGQAYVRKDGEWVLLSTFL (SEQ ID NO: 35).

The foldon domain is located at the C-terminus of the recombinant RSV F protein of the present disclosure and may be linked directly or via a linker. Examples of the amino acid linker contain, but are not limited to, short amino acid linkers such as GG, GS, and SAIG, or longer amino acid linkers comprising the repeating sequence GG.

In another embodiment, the recombinant RSV F protein of the present disclosure may be derived from the F protein of RSV subtype A, the F protein of RSV subtype B, or the bovine RSV F protein. In addition, the recombinant RSV F protein of the present disclosure may have the F2 polypeptide and the F1 polypeptide linked directly or via a linker.

The present invention also provides an isolated nucleic acid molecule encoding the recombinant RSV F protein.

The term "nucleic acid," "nucleic acid molecule," or "nucleic acid sequence" refers to a DNA (cDNA or genomic DNA) or RNA molecule or sequence, and "nucleic acid composition" refers to a composition comprising such a nucleic acid, nucleic acid molecule, or nucleic acid sequence. The RNA may be mRNA, viral RNA, self-replicating RNA, circular RNA, or replicon RNA. In a preferred embodiment, the RNA may be mRNA. The RNA, preferably mRNA, may further comprise at least one selected from a cap structure, a poly(A) sequence, a poly(C) sequence, a histone-stem loop, and/or a 3'-terminal sequence element.

In one embodiment, the nucleic acid molecule may encode a recombinant precursor polypeptide of the RSV F protein. In addition, the precursor polypeptide may comprise, in order from the N-terminus to the C-terminus, a signal peptide, an F2 polypeptide, a p27 polypeptide, an extracellular domain of the F1 polypeptide, and a trimerization domain.

Optionally, the precursor polypeptide may further comprise a protease cleavage site and a tag for purification at the C-terminus of the trimerization domain. Examples of the protease cleavage site contain, but are not limited to, a thrombin cleavage site (LVPRGS) recognized by thrombin. Examples of the tag for purification contain, but are not limited to, a histidine-tag (HHHHHH), a strep tag II (WSHPQFEK), or a c-tag (EPEA).

As described above, the wild-type precursor polypeptide typically comprises, in order from the N-terminus to the C-terminus, a signal peptide (residues 1-25), an F2 polypeptide (residues 26-109), a p27 polypeptide (residues 110-136), and an F1 polypeptide (residues 137-574). Upon translation, the signal peptide is removed, and the remaining portion of the F0 precursor (i.e., residues 26-574) is further cleaved by a cellular protease (furin) to remove the p27 polypeptide (residues 110-136), resulting in the formation of the RSV F protomer, which is a heterodimer in which the F1 and F2 polypeptides are linked together via two disulfide bonds; finally, the resulting recombinant F protein is in the form of a trimer in which three protomers are assembled.

Therefore, the nucleic acid molecule of the present disclosure may encode a recombinant precursor polypeptide of an RSV F protein, and the recombinant F protein finally expressed therefrom may not comprise, in the configuration of the precursor polypeptide described above, a signal peptide, a p27 polypeptide, a protease cleavage site, a tag, or the like.

The nucleic acid molecule may be cloned using conventional molecular biology techniques or newly generated by DNA synthesis using known recombinant techniques.

The present invention also provides a vector and an isolated host cell comprising the nucleic acid molecule described above.

To facilitate replication and expression, the nucleic acid molecule of the present disclosure may be integrated into a vector, such as an expression vector. The term "vector" refers to a genetic construct comprising essential regulatory elements operably linked so that a gene insert encoding a target protein is expressed within a cell of an individual, and various types of vectors such as plasmids, viral vectors, bacteriophage vectors, cosmid vectors, and the like may be used. Such vectors may be easily manipulated by methods well known to those of ordinary skill in the art and may be designed, for example, to be replicable in prokaryotic cells and/or eukaryotic cells.

Host cells for producing the recombinant RSV F protein contain prokaryotic (i.e., bacterial) host cells, such as E. coli, or eukaryotic host cells. Preferred host cells are eukaryotic host cells, which may contain, but are not limited to, insect cells, such as Spodoptera frugiperda (Sf) cells such as Sf9 and Sf21 using a Baculovirus expression system, Trichoplusia ni cells such as High Five (Hi-5) cells and Drosophila S2 cells, and mammalian cells such as Chinese hamster ovary (CHO) cells, such as CHO K1, CHO pro3-, CHO DG44, CHO P12, and the like.

The present disclosure also provides a method for producing a recombinant RSV F protein, comprising the steps of: introducing the nucleic acid molecule into an isolated host cell; and obtaining the recombinant RSV F protein by culturing the host cell.

Introducing the nucleic acid molecule into a cell may be performed using suitable standard techniques as known in the art, for example, transfection, electroporation, electroinjection, microinjection, calcium phosphate co-precipitation, the calcium chloride/rubidium chloride method, retroviral infection, DEAE-dextran, the cationic liposome method, polyethylene glycol-mediated uptake, gene gun, and the like, but is not limited thereto.

Cell culture medium is available from various sources, and a suitable medium may typically be selected so that the host cell expresses the protein of interest, the recombinant RSV F protein herein. Suitable media may or may not comprise serum. Culture conditions, such as temperature, pH, and the like, are those previously used with the host cell selected for expression and will be apparent to one of ordinary skill in the related art.

The secreted recombinant RSV F protein is recovered from the culture medium. For example, cells may be harvested by depth filtration using primary and secondary filters, centrifugation, or the like, and disrupted by physical or chemical means or the like to generate a retained crude extract for further purification. Cells used for expression of the protein may be disrupted by any convenient method, including freeze-thaw cycling, sonication, mechanical disruption, or the use of cell lysing agents, or other methods well known to those of ordinary skill in the related art.

The recombinant RSV F protein may be finally obtained by a purification process comprising a chromatographic purification step. Suitable methods for purification of the target protein are known in the art, including precipitation and various types of chromatography, such as hydrophobic interaction, ion exchange, affinity, chelation, and size exclusion.

The recombinant RSV F protein provided herein may be used as an active ingredient in an immunogenic composition against RSV infection. Accordingly, the present disclosure also provides an immunogenic composition comprising the recombinant RSV F protein as described above, or a nucleic acid molecule encoding the recombinant RSV F protein. The immunogenic composition may be a vaccine composition.

The term "vaccine" refers to an immunogenic composition for inducing an immune response to reduce or prevent the risk of a disease or infection, or to improve or treat an existing disease or infection. The vaccine may be multivalent. The vaccine may be used as a therapeutic or prophylactic agent.

The composition of the present disclosure may simultaneously induce humoral immunity and cellular immunity in an individual. The composition of the present disclosure may elicit antigen-specific T cell responses and/or B cell responses.

The term "immune response" refers to a change in the activity of cells of the immune system, such as B cells, T cells, or monocytes, as a result of stimulation directly or indirectly through cellular or cytokine mediation. The immune response may be a specific (T cell and/or B cell) and/or non-specific immune response.

Without limiting the action of the present invention in any way, delivery of a composition according to the present invention is useful for inducing an immune response, for example, inducing a T cell response, such as a CD4+ T cell response or a CD8+ T cell response to an antigen, or inducing a B cell response. A CD4+ T cell response and a CD8+ T cell response may occur together with or independently of a humoral response or other specific or non-specific immune responses.

The composition of the present disclosure may be used for the prevention and/or treatment of an RSV infection or a disease caused by an RSV infection. In certain embodiments, the prevention and/or treatment may target patient groups susceptible to an RSV infection. Such target groups contain, but are not limited to, for example, the elderly (e.g., ≥ 50 years, ≥ 60 years, and preferably ≥ 65 years), adolescents (e.g., ≤ 5 years, ≤ 1 year), pregnant women (for maternal immunization), hospitalized patients, and patients who have been treated with antiviral compounds but exhibit an inadequate antiviral response.

The vaccine composition of the present disclosure may be a subunit vaccine comprising a recombinant RSV F protein or an RNA vaccine comprising an RNA nucleic acid molecule encoding a recombinant RSV F protein. In a preferred embodiment, the RNA may be mRNA. The RNA, preferably mRNA, may further comprise at least one selected from a cap structure, a poly(A) sequence, a poly(C) sequence, a histone-stem loop, and/or a 3'-terminal sequence element.

In one embodiment, the RNA nucleic acid molecule may comprise a chemical modification.

In a preferred example, the chemical modification may be at least one selected from the group consisting of pseudouridine, N1-methylpseudouridine, N1-ethylpseudouridine, 2-thiouridine, 4'-thiouridine, 5-methylcytosine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine, and 2'-O-methyl uridine.

The chemical modification may occur at the 5-position of uracil. For example, 5% or more, 10% or more, 25% or more, 50% or more, 80% or more, 90% or more, or 100% of uracils in the RNA nucleic acid molecule may be replaced with a modified uracil (e.g., 5-substituted uracil), but is not limited thereto.

Other examples of chemical modification may contain 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentyladenosine, N6-(cis-hydroxyisopentyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, or N2,N2-dimethyl-6-thio-guanosine, but the present disclosure is not limited thereto.

In one embodiment, the RNA nucleic acid molecule may be produced through *in vitro* transcription.

*In vitro* transcription of RNA is known in the art (see, for example, International Publication WO2014/152027). For example, for *in vitro* transcription, a linearized DNA template, nucleotide triphosphates, RNA polymerase, a capping enzyme, a ribonuclease inhibitor for preventing RNase contamination, a pyrophosphatase for destroying pyrophosphate that interferes with transcription, MgCl₂, a suitable pH buffer, and the like are required. For example, the RNA nucleic acid molecule may be generated using a non-amplified, linearized DNA template in an *in vitro* transcription reaction. The DNA template may be isolated DNA or cDNA, and may comprise an RNA polymerase promoter, for example, a T7 promoter. As the RNA polymerase, a phage RNA polymerase, for example, T7 RNA polymerase, T3 RNA polymerase, SP6 RNA polymerase, or a variant thereof may be used. The generated RNA nucleic acid molecule may be capped via enzymatic capping and may be purified by a chromatographic method, for example, use of an oligo dT substrate.

In one embodiment, the RNA nucleic acid molecule herein may be formulated in a lipid nanoparticle.

In some embodiments, the lipid nanoparticle may have an average diameter of 1 nm or more, 2 nm or more, 3 nm or more, 4 nm or more, 5 nm or more, 6 nm or more, 7 nm or more, 8 nm or more, or 9 nm or more, 10 nm or more, 20 nm or more, 30 nm or more, 40 nm or more, 50 nm or more, 60 nm or more, 70 nm or more, 80 nm or more, or 90 nm or more, and 100 nm or less, 200 nm or less, 300 nm or less, 400 nm or less, 500 nm or less, 600 nm or less, 700 nm or less, 800 nm or less, or 900 nm or less, but is not limited thereto.

Formation of lipid nanoparticles may be achieved by methods known in the art and/or as described in US Patent Publication No. US 20120178702. In one embodiment, the lipid nanoparticle may comprise a cationic lipid, a non-cationic lipid, a PEG-modified lipid, or a sterol. More specifically, the lipid nanoparticle may comprise DLin-MC3-DMA, DLin-DMA, DLin-D-DMA, DLin-K-DMA, C12-200, DLin-KC2-DMA, 98N12-5, DODMA, PLGA, polyethylene glycol (PEG), PEG-DMG, PEG2000-DMG, pegylated lipids, amino alcohol lipids, cholesterol, or 1,2-distearoyl-sn-glycero-3-phosphocholine (DSPC), but is not limited thereto.

As a non-limiting example, the polycation may comprise cationic peptides or polypeptides such as, but not limited to, polylysine, polyornithine, and/or polyarginine, which are described in International Publication No. WO2012013326 or US Patent Publication No. US 20130142818. In another example, the RNA nucleic acid molecule herein may be formulated in a lipid nanoparticle comprising a non-cationic lipid such as, but not limited to, cholesterol or dioleoyl phosphatidylethanolamine (DOPE). Lipid nanoparticle formulations may be affected by, but not limited to, the choice of the cationic lipid component, the degree of cationic lipid saturation, the nature of pegylation, the ratio of all components, and biophysical parameters such as size.

In another embodiment, the RNA nucleic acid molecule may also be formulated in a liposome.

In some embodiments, the liposome may be a DiLa2 liposome (Marina Biotech, Bothell, WA), SMARTICLES^{®} (Marina Biotech, Bothell, WA), a neutral DOPC (1,2-dioleoyl-sn-glycero-3-phosphocholine)-based liposome, a hyaluronan-coated liposome (Quiet Therapeutics, Israel), or a lyophilized gel-phase liposome (US Patent Publication No. US2012060293), but is not limited thereto.

Liposomes comprise phospholipids, for example, phosphatidylcholines that may be non-crystalline at room temperature, for example, egg yolk phosphatidylcholine, dioleoyl phosphatidylcholine, or dilauryl phosphatidylcholine, and suitably, may be dioleoyl phosphatidylcholine (DOPC), but are not limited thereto. Liposomes may also comprise a sterol. Sterols are selected from the group consisting of β-sitosterol, stigmasterol, ergosterol, ergocalciferol, and cholesterol, and are known in the art.

The composition herein may further comprise a pharmaceutically acceptable carrier, additive, excipient, diluent, and/or adjuvant. Other examples of carriers contain colloidal silicon oxide, magnesium stearate, cellulose, and sodium lauryl sulfate, and other knowledge known in the art, for example, Remington's Pharmaceutical Sciences, may be referred to. Adjuvants contain (1) aluminum salts (e.g., aluminum hydroxide, aluminum phosphate, aluminum sulfate, etc.), (2) oil-in-water emulsion formulations (with or without specific immunostimulants such as muramyl peptides or bacterial cell wall components), for example, (a) MF59 (WO90/14837) comprising 5% squalene, 0.5% Tween 80, and 0.5% Span 85 formulated into submicron particles (optionally, although not essentially, containing various amounts of N-acetylmuramyl-L-alanyl-D-isoglutaminyl-L-alanine-2-(1',2'-dipalmitoyl-sn-glycero-3-hydroxyphosphoryloxy)-ethylamine (MTP-PE)), (b) SAF comprising 10% squalene, 0.4% Tween 80, 5% pluronic-block polymer, and N-acetyl-muramyl-L-threonyl-D-isoglutamine (thr-MDP), microfluidized into submicron particles or agitated to generate a large particle size emulsion, and (c) RibiTM adjuvant system (RAS) comprising one or more bacterial cell wall components selected from the group consisting of monophosphoryl lipid A (MPL), trehalose dimycolate (TDM), and cell wall skeleton (CWS), 2% squalene, and 0.2% Tween 80; (3) saponin adjuvants, for example, Quil A or STIMULON^{™} QS-21 (Antigenics, Framingham, MA); (4) Freund's complete adjuvant (CFA) and incomplete adjuvant (IFA); (5) cytokines, for example, interleukins (e.g., IL-1, IL-2, IL-4, IL-5, IL-6, IL-7, IL-12, etc.), interferons (e.g., gamma interferon), macrophage colony stimulating factor (M-CSF), tumor necrosis factor (TNF), etc.; (6) bacterial ADP-ribosylating toxins, for example, cholera toxin (CT), pertussis toxin (PT), or heat-labile toxin (LT) of E. coli, especially detoxified mutants of LT-R72, CT-S109, and PT-K9/G129 (WO93/13302 and WO92/19265); and (7) other substances acting as immunostimulants that enhance the effectiveness of vaccines, but the composition is not limited thereto.

The composition may be administered using standard routes of administration. Such routes of administration may contain, but are not limited to, intramuscular, intranasal, intradermal, subcutaneous, intraarticular, intravenous, intrasplenic, intrasynovial, intrasternal, intrathecal, intrahepatic, intralesional, intracranial, transdermal, intrapulmonary, intraperitoneal, intracardiac, intraarterial, sublingual, or intralymphatic routes. In addition, the composition herein may efficiently deliver RNA into the body using antigen presenting cells, a gene gun, or electroporation, but is not limited thereto. The administration regimen may be a single dose or multiple dose schedule.

The term "effective amount" refers to an amount sufficient to achieve a desired result when administered to an individual, including a human, for example, an amount effective for treating or preventing an RSV infection. The effective amount may be determined based on the target tissue, target cell type, means of administration, size and degree of modification of the RNA nucleic acid molecule, physical characteristics of other components, age, weight, and condition of the individual, and the like. The dosage or treatment regimen may be adjusted to provide the optimal therapeutic response as understood by those of ordinary skill in the art.

### [Mode for Carrying Out the Invention]

Hereinafter, the present invention will be described in more detail with reference to the following examples. However, these are only for illustrating the present invention, and the scope of the present invention is not limited by these examples. It is obvious to those skilled in the art that the examples described below may be modified without departing from the essential spirit of the invention.

### Summary

Substitution of amino acid residues 486-488 from DEF to LLW may improve the structural stability of the RSV trimeric fusion protein, which is referred to as an 'SK mutation'. In this study, we focused on developing and evaluating prefusion RSV F proteins stabilized by introducing the SK mutation, especially DS-Cav1 and DS2 variants. In this study, both subunit and mRNA vaccine platforms were evaluated. In in-silico and *in vitro* analyses, the SK mutation significantly improved protein stability and increased neutralizing antibody titers. In immunogenicity testing in an animal model, DS-Cav1 SK elicited a significantly high neutralizing antibody response, whereas DS2 variants, especially DS2-3 and DS2-4, showed higher levels of neutralizing antibodies. In addition, although combining various antigen strains may improve cross-reactivity, the optimal dosage and combination are essential to achieve balanced and robust protection against multiple RSV strains.

### Example 1. in vitro thermostability test

50 µL of 4 µg/mL DS-Cav1 and DS-Cav1 SK from A2 and B18537 strains were dispensed and heat-treated for 30 minutes using a PCR device. ELISA analysis was performed to analyze the fraction of the trimeric prefusion form. The heat-treated samples were diluted to 2 µg/mL with a sample coating buffer (1X PBS), added to nickel-coated plates, and incubated overnight at 4°C. The captured proteins were sequentially detected with an AM14 antibody (anti-F (RSV) AM14 human Fc, 1:2,000) and a secondary antibody (goat anti-human IgG Fc cross-adsorbed secondary antibody, HRP, 1:10,000). The plates were washed, and 100 µL of TMB substrate was added to each well, followed by incubation at room temperature for 10 minutes in the dark. 100 µL of TMB stop solution was added to each well to terminate the reaction, and the absorbance was measured by selecting two wavelengths (measurement wavelength: 450 nm, reference wavelength: 650 nm) using a microplate reader.

### Example 2. Immunogenicity study of RSV vaccine candidates

To evaluate the immunogenicity of RSV vaccine candidates using 6-week-old female BALB/c mice, the candidates were administered intramuscularly to the mice twice at week 0 and week 3. Serum was collected at week 6 (3 weeks after the last immunization) to measure neutralizing antibody levels.

**[Table 2]**

| comparison of immunogenicity of DS-Cav1 and DS-Cav1 SK candidates (Set 1) | | | | | | |
|---|---|---|---|---|---|---|
| **Group #** | **platform** | **strain** | **antigen** | **dosage (µg/head)** | **adjuvant** | **mouse #** |
| 1 | | A2 | DS-Cav1 | | | |
| 2 | subunit | | DS-Cav1 SK | 5 | AddaVax | 10 |
| 3 | | B18537 | DS-Cav1 | | | |
| 4 | | | DS-Cav1 SK | | | |

**[Table 3]**

| Immunogenicity of subunit vaccine candidates (Set 2) | | | | | | |
|---|---|---|---|---|---|---|
| **Group #** | **platform** | **strain** | **antigen** | **dosage (µg/head)** | **adjuvant** | **mouse #** |
| 1 | subunit | A2 | DS-Cav1 | 5 | AddaVax | 10 |
| 2 | | | DS-Cav1 SK | | | |
| 3 | | | DS2-1 | | | |
| 4 | | B18537 | DS2-1 SK | | | |
| 5 | | | DS2-2 | | | |
| 6 | | | DS2-2 SK | | | |
| 7 | | | DS2-3 | | | |
| 8 | | | DS2-3 SK | | | |
| 9 | | | DS2-4 | | | |
| 10 | | | DS2-4 SK | | | |
| 11 | | | DS-Cav1 | | | |
| 12 | | | DS-Cav1 SK | | | |
| 13 | | | DS2-1 | | | |
| 14 | | | DS2-1 SK | | | |
| 15 | | | DS2-2 | | | |
| 16 | | | DS2-2 SK | | | |
| 17 | | | DS2-3 | | | |
| 18 | | | DS2-3 SK | | | |
| 19 | | | DS2-4 | | | |
| 20 | | | DS2-4 SK | | | |

**[Table 4]**

| evaluation of cross-neutralization efficacy of monovalent/bivalent subunit vaccine candidates against A2 and B18537 strains (Set 3) | | | | | | |
|---|---|---|---|---|---|---|
| **Group #** | **platform** | **strain** | **antigen** | **dosage (µg/head)** | **adjuvant** | **mouse #** |
| 1 | subunit | A2 | DS2-4 | 0.2 | AddaVax | 10 |
| 2 | | | | 1 | | |
| 3 | | | | 5 | | |
| 4 | | B18537 | | 0.2 | | |
| 5 | | | | 1 | | |
| 6 | | | | 5 | | |
| 7 | | A2 DS2-4 + B18537 DS2-4 | | 0.1 + 0.1 | | |
| 8 | | | | 0.5 + 0.5 | | |
| 9 | | | | 2.5 + 2.5 | | |

**[Table 5]**

| Immunogenicity of mRNA vaccine candidates (Set 4) | | | | | | |
|---|---|---|---|---|---|---|
| **Group #** | **platform** | **strain** | **antigen** | **dosage (µg/head)** | **LNP** | **mouse #** |
| 1 | mRNA | A2 | DS-Cav1 | 5 | ALC-0315 | 10 |
| 2 | | | DS-Cav1 SK | | | |
| 3 | | | DS-Cav1 | | | |
| 4 | | | DS-Cav1 SK | | | |
| 5 | | | DS2-1 | | | |
| 6 | | | DS2-1 SK | | | |
| 7 | | | DS2-2 | | | |
| 8 | | | DS2-2 SK | | | |
| 9 | | | DS2-3 | | | |
| 10 | | | DS2-3 SK | | | |
| 11 | | | DS2-4 | | | |
| 12 | | | DS2-4 SK | | | |
| 13 | | | WT | | | |
| 14 | | | WT SK | | | |

### Example 3. serum binding assay (ELISA)

An antigen (RSV-preF protein) was diluted to 1 µg/mL using sample coating buffer (1X PBS), dispensed into a 96-well plate, sealed with sealing tape, and incubated overnight at 4°C. The coated plate was washed three times with 300 µL of washing buffer (0.05% Tween-20 in PBS), then each well was blocked with 250 µL of blocking buffer (2% skim milk in PBS), sealed with sealing tape, and incubated at RT for 1 hour. Before the blocking step was completed, cluster tubes and sample dilution buffer (2% skim milk and 0.05% Tween-20 in PBS) were prepared to dilute the samples. Samples were serially diluted 5-fold starting from 1:50. After the blocking step, the plate was washed, and 50 µL per well of the diluted serum was dispensed into the blocked plate. After dispensing, sealing tape was applied, and the plate was incubated in a 37°C incubator for 1 hour. 100 µL of diluted secondary antibody (goat antimouse IgG, human ads-HRP, 1:2,000) was dispensed into each well. After dispensing, sealing tape was applied, the plate was covered with foil, and incubated in a 37°C incubator for 1 hour. The plate was washed, and 100 µL of TMB substrate was added to each well, followed by incubation at room temperature for 10 minutes in the dark. 100 µL of TMB stop solution was added to each well to terminate the reaction, and absorbance was measured using a microplate reader at two wavelengths (measurement wavelength: 450 nm; reference wavelength: 650 nm).

### Example 4. serum neutralization assay (FRNT)

One day before virus infection, 4 x 10⁴ A549 cells/well were prepared in a 96-deep well plate. Collected serum was subjected to heat inactivation at 56°C for 30 minutes. Serum samples were diluted with 7 additional 3-fold serial dilutions, mixed with virus (RSV-A2-P5-230724) at a 1:1 ratio, and incubated at 37°C for 1 hour. The initial dilution factors for serum and virus were 1/10 and 1/40 (400 FFU/100 µL), respectively, in MEM containing 2% FBS. A549 cells in a 96-well plate were treated with the mixture of serum and virus and incubated at 37°C for 2 hours. The cells were overlaid with 0.8% methyl cellulose in MEM containing 2% FBS and incubated at 37°C for 48 hours. After 48 hours, cells were fixed with methanol at 4°C for 24 hours. RSV F protein expressed in the infected cells was captured, and then hRSV-F antibody (1:2,000) and diluted secondary antibody (goat anti-rabbit IgG(H+L)-HRP, 1:5,000) were sequentially bound. TrueBlue substrate was added to each well, and incubation was performed at room temperature for 20 minutes. Foci were counted with a Cytation 7 cell imaging multi-mode reader. Results were analyzed using a non-linear regression curve fitting method, and a 50% effective concentration (EC₅₀) was calculated.

### Experimental Results

### 1. Development of stabilized prefusion RSV F protein through introduction of SK mutation

Substitution of amino acid residues 486-488 from DEF to LLW may improve the structural stability of the RSV trimeric fusion protein; this modification is referred to as the 'SK mutation.' In this study, we performed *in silico* analysis to evaluate changes in the structural stability and interactions of the RSV fusion protein following the SK mutation. In addition, the stability of the protein and its immunogenicity were evaluated *in vivo* using an animal immunization model.

### 1-1. in silico analysis

*In silico* analysis was performed to closely examine the structural characteristics of the RSV F protein. Homology modeling of DS-Cav1 was performed using the SWISS-MODEL server with a template structure of bovine RSV F (Protein Data Bank entry, 5TDG). Binding energies and residues were calculated using the MODELLER module of the Discovery Studio 2024 application (BIOVIA, San Diego, CA, USA).

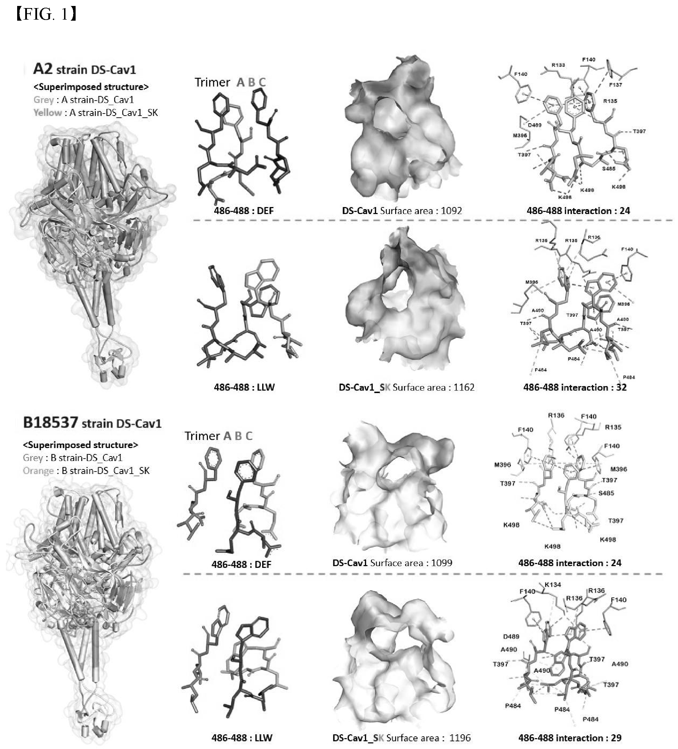

Substitution of amino acid residues 486-488 from DEF to LLW in the DS-Cav1 prefusion F protein results in the formation of a tryptophan cluster at the subunit interface. In addition, leucine substitution at that site reduces negative charge repulsion between subunits of the RSV F trimer. This effect enhances hydrophobic interaction, potentially increasing structural stability and making subunit binding stronger, which ultimately may lead to the formation of a potentially stabilized trimeric structure (FIG. 1).

According to the *in silico* analysis, LLW substitution reduced the potential energy of the DS-Cav1 SK prefusion F protein (A2 strain) from -60,601 kcal/mol to -62,971 kcal/mol, indicating that the structure is more stable. In the case of the B18537 strain, the potential energy slightly increased from -63,324 kcal/mol to -62,855 kcal/mol, suggesting that the stability of this strain slightly decreased. In DS-Cav1 SK, the number of residues interacting with positions 486-488 significantly increased. The number of interacting residues increased from 24 to 32 for the A2 strain and from 24 to 29 for the B18537 strain. This indicates that the interactions were strengthened and the subunit binding became stronger due to the LLW substitution (Table 6).

**[Table 6]**

| *in silico* analysis of RSV prefusion F protein stability | | | | | | | |
|---|---|---|---|---|---|---|---|
| **strain** | **RSV F subunit** | **potential energy (kcal/mol)** | **van der Waals energy (kcal/mol)** | **electrostatic energy (kcal/mol)** | **number of interacting residues with 446-448 contact** | **surface area** | **number of unfavorable interactions** |
| **A2** strain | **DS-Cav1** | -60,601 | -10,878 | -56,486 | 24 | 1,092 | 4 |
| | **DS-Cav1 SK** | -62,971 | -11,468 | -61,155 | 32 | 1,162 | 3 |
| **B18537** strain | **DS-Cav1** | -63,324 | -11,913 | -61,233 | 24 | 1,099 | 6 |
| | **DS-Cav1 SK** | -62,855 | -11,692 | -60,956 | 29 | 1,196 | 4 |

### 1-2. in vitro thermal stability

The increase in stability observed in the *in silico* analysis was confirmed by an *in vitro* thermostability test. To investigate the thermostability of the engineered prefusion RSV F proteins (DS-Cav1 and DS-Cav1 SK), the AM14 (Site IV/V) antibody, which is widely used to measure the trimeric prefusion form, was used.

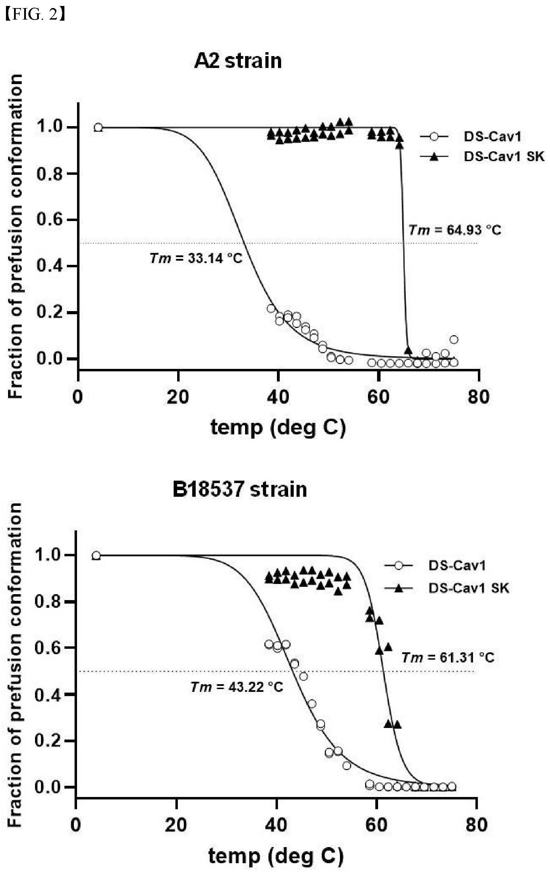

As a result of the *in vitro* thermostability test, when residues 486-LLW-488 were substituted, the melting temperature of the trimeric structure significantly increased. The melting temperature of DS-Cav1 was 33.14°C, whereas DS-Cav1 SK had a significantly higher melting temperature of 64.93°C in the A2 strain. Similarly, for the B18537 strain, the melting temperature increased from 43.22°C to 61.31°C (FIG. 2). These results support the hypothesis that enhanced hydrophobic interaction and tryptophan clustering at the subunit interface contribute to a thermally more stable trimeric structure.

### 1-3. Immunogenicity of engineered prefusion RSV F proteins

To evaluate prefusion RSV F proteins in which the LLW substitution was introduced, the immunogenicity of DS-Cav1 SK proteins (A2 and B18537) was evaluated in an animal model and compared with the immunogenicity of DS-Cav1 proteins. Mice were immunized by intramuscular administration of 5 µg of RSV F at week 0 and week 3 using AddaVax as an adjuvant. At week 6, animals were bled and RSV neutralizing antibody titers in serum were determined.

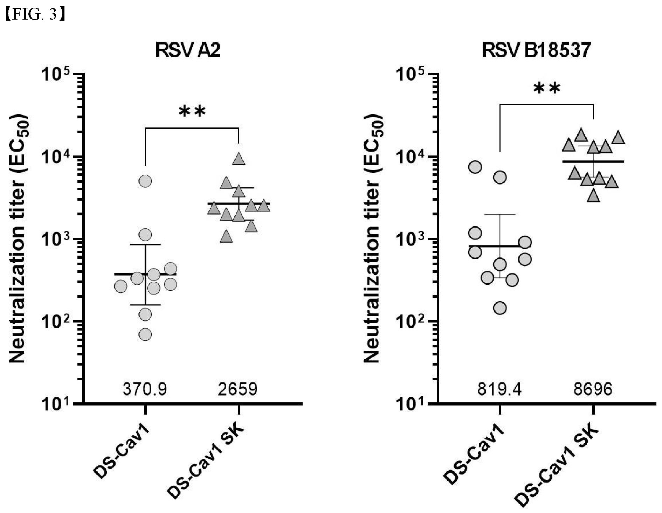

The DS-Cav1 SK protein significantly improved the ability to induce neutralizing antibodies in mice. This improvement was observed in both the A2 (7.17-fold) and B18537 (10.61-fold) strains compared to the corresponding DS-Cav1 strains (FIG. 3).

In conclusion, substitution of residues 486-488 from DEF to LLW in the DS-Cav1 prefusion RSV F protein (DS-Cav1 SK) results in the formation of a hydrophobic cluster, which strengthens subunit interaction and increases structural stability. This is evidenced by the lower potential energy and greater number of interacting residues in DS-Cav1 SK. The increase in stability is further confirmed by the higher melting temperatures observed in the thermal stability test, which correlate with a significantly higher neutralizing antibody response in the immunized mice. Such structural stability leads to improved immunogenicity, suggesting that DS-Cav1 SK has potential for use as a more stable and immunogenic vaccine candidate against RSV.

### 2. Selection of subunit vaccine candidates

DS-Cav1 comprises a prefusion stabilized form of the fusion glycoprotein and elicits a strong protective response against RSV. DS2 variants comprise genetically linked F subunits, a deleted fusion peptide, and interprotomer movement stabilized by additional disulfide bonds and specific mutations. RSV F proteins including DS-Cav1, DS2 variants, and the SK mutation (PRT-R03859) were successfully produced. Information on the prefusion RSV F protein constructs is shown in Table 7 and Table 8. In this study, we evaluated the immunogenicity and protective efficacy of these stabilized F RSV vaccine candidates through an animal immunization model.

### 2-1. RSV F vaccine candidate construct

**[Table 7]**

| RSV F vaccine candidate construct list | | |
|---|---|---|
| **antigen (A2 strain)** | **construct** | **SEQ ID NO** |
| DS-Cav1 | DS-Cav1 | 2 |
| DS-Cav1 SK | DS-Cav1 D486L E487L F488W | 3 |
| DS2-1 | Sc9-10 DS-Cav1 | 4 |
| DS2-1 SK | Sc9-10 DS-Cav1 D486L E487L F488W | 5 |
| DS2-2 | Sc9-10 DS-Cav1 A149C Y458C | 6 |
| DS2-2 SK | Sc9-10 DS-Cav1 A149C Y458C D486L E487L F488W | 7 |
| DS2-3 | Sc9-10 DS-Cav1 N183GC N428C | 8 |
| DS2-3 SK | Sc9-10 DS-Cav1 N183GC N428C D486L E487L F488W | 9 |
| DS2-4 | Sc9-10 DS-Cav1 A149C Y458C S46G E92D S215P K465Q | 10 |
| DS2-4 SK | Sc9-10 DS-Cav1 A149C Y458C S46G E92D S215P K465Q D486L E487L F488W | 11 |

**[Table 8]**

| RSV F vaccine candidate construct list | | |
|---|---|---|
| **antigen (B18537 strain)** | **construct** | **SEQ ID NO** |
| DS-Cav1 | DS-Cav1 | 12 |

| **antigen (B18537 strain)** | **construct** | **SEQ ID NO** |
|---|---|---|
| DS-Cav1 SK | DS-Cav1 D486L E487L F488W | 13 |
| DS2-1 | Sc9-10 DS-Cav1 | 14 |
| DS2-1 SK | Sc9-10 DS-Cav1 D486L E487L F488W | 15 |
| DS2-2 | Sc9-10 DS-Cav1 A149C Y458C | 16 |
| DS2-2 SK | Sc9-10 DS-Cav1 A149C Y458C D486L E487L F488W | 17 |
| DS2-3 | Sc9-10 DS-Cav1 N183GC N428C | 18 |
| DS2-3 SK | Sc9-10 DS-Cav1 N183GC N428C D486L E487L F488W | 19 |
| DS2-4 | Sc9-10 DS-Cav1 A149C Y458C S46G E92D S215P K465Q | 20 |
| DS2-4 SK | Sc9-10 DS-Cav1 A149C Y458C S46G E92D S215P K465Q D486L E487L F488W | 21 |

### 2-2. Immunogenicity of prefusion RSV F subunit vaccine candidates

To evaluate the immunogenicity of DS-Cav1, DS2-1, DS2-2, DS2-3, and DS2-4 prefusion RSV F proteins (A2 and B18537), evaluation was performed in an animal model. Mice were immunized by intramuscular administration of 5 µg of RSV F protein at week 0 and week 3 using AddaVax as an adjuvant. At week 6, animals were bled to determine serum prefusion F-specific IgG titers and RSV neutralizing antibody titers.

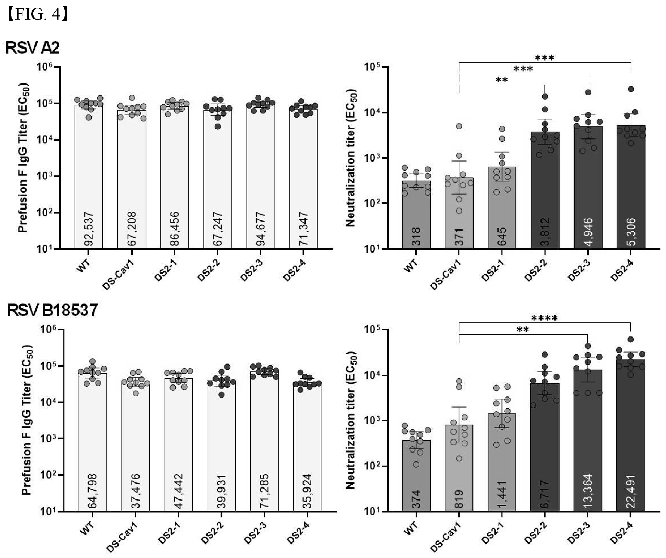

All mice produced antibody titers against the RSV Fusion protein as measured by ELISA. The DS2 variants induced significantly higher neutralizing antibody titers against the RSV A2 virus strain compared to DS-Cav1 and the wild-type. Compared to the DS-Cav1 Group, the significant increase in neutralizing antibody titers observed in the DS2-2 (10.30-fold), DS2-3 (13.36-fold), and DS2-4 (14.34-fold) Groups suggests that these variants have improved immunogenic properties. Similarly, in the case of the RSV B18537 virus strain, the DS2-3 (16.32-fold) and DS2-4 (27.46-fold) Groups were observed to significantly increase neutralizing antibody titers compared to the DS-Cav1 group (FIG. 4 and Table 9). Through neutralizing antibody responses in both RSV A2 and B18537 virus strains, it was confirmed that the DS2-3 and DS2-4 candidates consistently performed better than other candidates and showed the highest efficacy in inducing neutralizing antibodies.

**[Table 9]**

| neutralizing antibody titers of RSV F subunit vaccine candidates | | | |
|---|---|---|---|
| **No.** | **candidate** | **A2 virus strain GMT (95% CI)** | **B18537 virus strain GMT (95% CI)** |
| 1 | wild-type (SEQ ID NO 22) | 318.2 (224.8 - 450.5) | 373.5 (240.7 - 579.6) |
| 2 | DS-Cav1 | 370.0 (160.0 - 859.7) | 819.4 (338.3 - 1,985) |
| 3 | DS2-1 | 645.4 (307.0 - 1,357) | 1,441 (695.3 - 2,987) |
| 4 | DS2-2 | 3,812 (1,998 - 7,272) | 6,717 (3,731 - 12,093) |
| 5 | DS2-3 | 4,946 (2,649 - 9,236) | 13,364 (7,081 - 25,222) |
| 6 | DS2-4 | 5,306 (2,998 - 9,388) | 22,491 (15,583 - 32,460) |

### 2-3. Immunogenicity of RSV F subunit vaccine candidates with SK mutation

In the previous experiment, the introduction of the SK mutation into the DS-Cav1 prefusion F protein resulted in improved protein structural stability and increased neutralizing antibody titers. To confirm whether a similar improvement in immunogenicity may be induced by introducing the SK mutation into the DS2 variants as well, the SK mutation was introduced into DS2-1, DS2-2, DS2-3, and DS2-4 to evaluate immunogenicity.

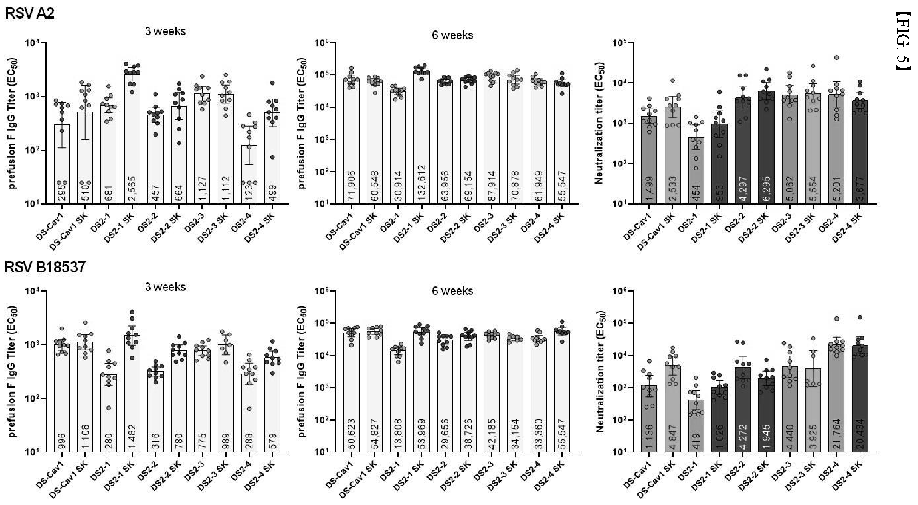

In the case of the A2 virus strain, the introduction of the SK mutation resulted in an approximately 1.69-fold increase in the neutralizing antibody titer of the DS-Cav1 SK group. In addition, the neutralizing antibody titers of the DS2-1, DS2-2, and DS2-3 Groups increased by about 2-fold, about 1.46-fold, and about 1.1-fold, respectively, after introduction of the SK mutation (FIG. 5 and Table 10).

In the case of the B18537 virus strain, the neutralizing antibody titer of the DS-Cav1 SK group increased by about 4.26-fold compared to DS-Cav1. In addition, the neutralizing antibody titer of the DS2-1 Group increased by about 2.4-fold after introduction of the SK mutation (FIG. 5 and Table 10).

These results indicate that immunogenicity is improved when the SK mutation is introduced into DS-Cav1 and DS2 variants. However, while the SK mutation significantly increased the immunogenicity of DS-Cav1, the effect on DS2 variants was more variable, possibly due to the impact of the SK mutation being reduced by the inherent structural stability of the DS2 variants. Nonetheless, DS2 variant vaccines, especially DS2 variant vaccines into which the SK mutation was introduced, still showed the potential to provide a superior protective effect against RSV, potentially providing a promising path for the development of more effective vaccines.

**[Table 10]**

| neutralizing antibody titers according to SK mutations in prefusion RSV F proteins | | | |
|---|---|---|---|
| **No.** | **Group** | **A2 virus strain GMT (95% CI)** | **B18537 virus strain GMT (95% CI)** |
| 1 | DS-Cav1 | 1,499 (978.6 - 2,296) | 1,136 (528.5 - 2,442) |
| 2 | DS-Cav1 SK | 2,533 (1,380 - 4,651) | 4,847 (2,474 - 9,495) |
| 3 | DS2-1 | 454.4 (227.8 - 906.4) | 419.4 (214.1 - 821.3) |
| 4 | DS2-1 SK | 953.1 (443.1 - 2,050) | 1,026 (629.9 - 1,673) |
| 5 | DS2-2 | 4,297 (2,289 - 8,064) | 4,272 (1,931 - 9,451) |
| 6 | DS2-2 SK | 6,295 (3,860 - 10,265) | 1,945 (1,151 - 3,285) |
| 7 | DS2-3 | 5,062 (2,891 - 8,865) | 4,440 (2,041 - 9,659) |
| 8 | DS2-3 SK | 5,554 (3,197 - 9,648) | 3,925 (1,093 - 14,098) |
| 9 | DS2-4 | 5,201 (2,521 - 10,731) | 21,764 (12,999 - 36,439) |
| 10 | DS2-4 SK | 3,677 (2,342 - 5,773) | 20,434 (11,110 - 37,582) |

### 3. Neutralizing antibody response and cross-reactivity of RSV antigen: evaluation of monovalent and bivalent antibodies

In RSV vaccine development, it is essential to combine various virus strains in order to achieve broad protection against various viral variants. Monovalent vaccines may provide strong protection against a target viral strain, but cross-reactivity against other virus strains is limited. Therefore, to develop a more effective vaccine, it is necessary to evaluate how various antigen combinations act to elicit neutralizing antibody responses against various virus strains. To address this need, an experiment was performed to evaluate the neutralizing antibody response by single or combined administration of prefusion RSV F candidates, including A2 and B18357 DS2-4 antigens. For the combination group, each virus strain antigen was mixed in equal amounts to constitute doses of 0.2, 1, and 5 µg.

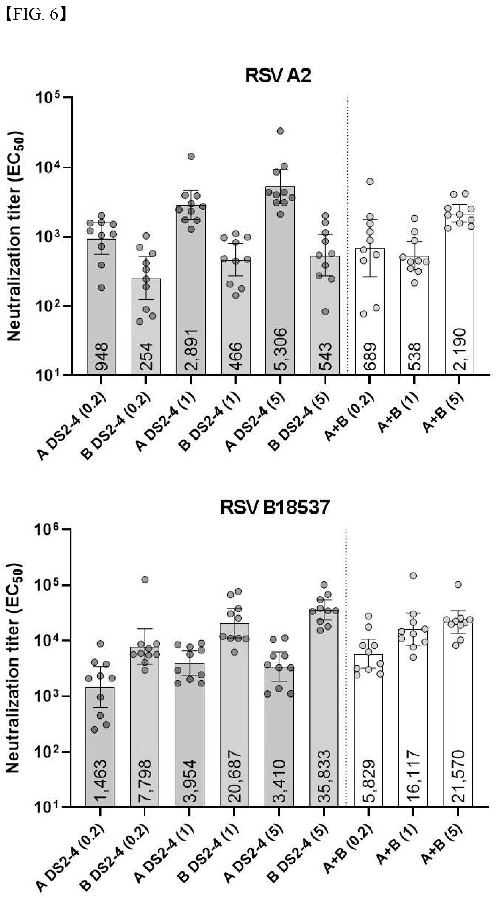

As a result, the A2 DS2-4 antigen alone showed a high neutralizing antibody titer against the RSV A2 virus strain, which increased in a dose-dependent manner to reach a peak of 5,306 (95% CI: 2,998 - 9,388) at the 5 µg dose. However, cross-reactivity against the RSV B18537 virus strain was at an intermediate level, with the highest titer being 3,954 (95% CI: 2,411 - 6,485) at 1 µg. When combined with the B18357 DS2-4 antigen, the response against the RSV A2 virus strain was higher than that of B18357 DS2-4 alone but was still lower than that of A2 DS2-4 alone, with the highest titer being 2,190 (95% CI: 1,640 - 2,924) at the 5 µg dose. On the other hand, the B18357 DS2-4 antigen alone showed limited cross-reactivity against the RSV A2 virus strain, with low neutralizing antibody titers across all doses, peaking at 543 (295% CI: 72.5 - 1,081) at the 5 µg dose. However, it provided strong protection against the RSV B18537 virus strain, showed a significant and dose-dependent response, and reached a maximum titer of 35,833 (95% CI: 23,507 - 54,622) at the 5 µg dose. When combined with the A2 DS2-4 antigen, the response against the RSV B18537 virus strain was improved compared to when B18357 DS-24 was administered alone, with a maximum titer of 21,570 (95% CI: 13,403-34,712) at the 5 µg dose, but this was lower than the peak titer achieved when B18357 DS2-4 was administered alone (FIG. 6 and Table 11).

In conclusion, the A2 DS2-4 antigen showed a high neutralizing antibody titer against the RSV A2 virus strain and intermediate cross-reactivity against the RSV B18537 virus strain, whereas the B18537 DS2-4 antigen showed a high neutralizing antibody titer against the RSV B18537 virus strain but limited cross-reactivity against the RSV A2 virus strain. For the combination in which A2 and B18537 DS2-4 antigens were mixed in equal amounts, the overall cross-reactivity was improved compared to B18537 DS2-4 alone, but did not exceed the individual efficacy of the A2 DS-24 antigen against the RSV A2 virus strain or the B18537 DS2-4 antigen against the RSV B18537 virus strain. These results suggest that although antigen combinations may improve the response, it is important to optimize the dose and combination to achieve balanced and robust protection against multiple RSV viral strains.

**[Table 11]**

| neutralizing antibody titers against RSV A2 and RSV B18537 virus strains: monovalent vs. bivalent antibody | | | |
|---|---|---|---|
| **No.** | **Group (antigen dose, µg)** | **RSV A2 FRNT GMT (95% CI)** | **RSV B18537 FRNT GMT (95% CI)** |
| 1 | A DS2-4 (0.2) | 948.0 (557.6 - 1,612) | 1,463 (623.6 - 3,432) |
| 2 | B DS2-4 (0.2) | 253.7 (124.8 - 515.6) | 7,798 (3,728 - 16,314) |
| 3 | A DS2-4 (1) | 2,891 (1,790 - 4,668) | 3,954 (2,411 - 6,485) |
| 4 | B DS2-4 (1) | 466 (271.4 - 800.9) | 20,687 (11,191 - 38,238) |
| 5 | A DS2-4 (5) | 5,306 (2,998 - 9,388) | 3,410 (1,862 - 6,246) |
| 6 | B DS2-4 (5) | 543 (272.5 - 1,081) | 35,833 (23,507 - 54,622) |
| 7 | A DS2-4 + B DS2-4 (0.2) | 688.9 (266.0 - 1785) | 5,829 (3,191 - 10,648) |
| 8 | A DS2-4 + B DS2-4 (1) | 537.8 (338.1 - 855.6) | 16,117 (8,287 - 31,345) |
| 9 | A DS2-4 + B DS2-4 (5) | 2,190 (1,640 - 2,924) | 21,570 (13,403 - 34,712) |

### 4. Selection of mRNA vaccine candidates

### 4-1. Immunogenicity of RSV F mRNA vaccine candidates

To compare the subunit and mRNA platforms, 12 candidates, including the wild-type, were developed as mRNA vaccines (REP-R02250, REP-R02445), and their immunogenicity was evaluated. While both platforms aim to elicit a strong immune response, the mRNA vaccine platform is characterized by, in particular, rapid development and strong immunogenicity.

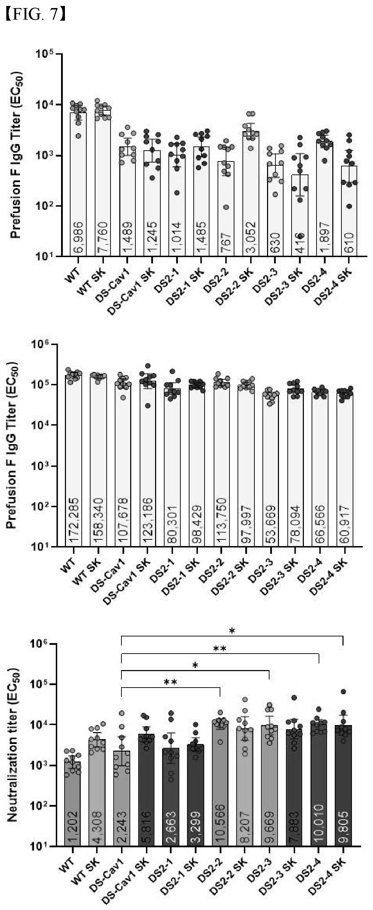

All tested mRNA vaccines successfully generated strong IgG responses against the RSV F protein. DS2 variant vaccines showed significantly higher neutralizing antibody levels compared to other Groups, which indicates a strong immune response and was consistent with the results observed in subunit platform experiments. DS2-1, DS2-2, DS2-3, and DS2-4 SK Groups all had significantly increased neutralizing antibody titers compared to the DS-Cav1 Group (FIG. 7).

The mRNA platform also demonstrated that the SK mutation has a significant impact on neutralizing antibody responses in various variants, expressed as fold change. In the case of the wild-type RSV F protein (WT), introduction of the SK mutation increased the neutralizing antibody titer by about 3.6-fold (from 1,202 to 4,308). In the DS-Cav1 Group, the SK mutation increased the titer by about 2.6-fold (from 2,243 to 5,816). This increase is attributed to the enhanced structural stability and thermal stability provided by the SK mutation. However, in DS2 variants, which are already stabilized antigens, the impact of the SK mutation was less significant, showing only modest changes in neutralizing antibody levels. These results suggest that while the SK mutation significantly increases immunogenicity in the wild-type and DS-Cav1 Groups, its impact on DS2 variants is relatively small. Nonetheless, DS2 variant vaccines, especially DS2 variant vaccines into which the SK mutation has been introduced, still demonstrated the potential to provide a superior protective effect against RSV, thereby providing a promising path for the development of more effective vaccines.

**[Table 12]**

| neutralizing antibody titers of RSV mRNA vaccine candidates | | | |
|---|---|---|---|
| **No.** | **Group** | **SEQ ID NO** | **RSV A2 FRNT GMT (95% CI)** |
| 1 | WT | 23 | 1,202 (841.7 - 1,715) |
| 2 | WT SK | 24 | 4,308 (2,868 - 6,472) |

| **No.** | **Group** | **SEQ ID NO** | **RSV A2 FRNT GMT (95% CI)** |
|---|---|---|---|
| 3 | DS-Cav1 | 25 | 2,243 (987.2 - 5,098) |
| 4 | DS-Cav1 SK | 26 | 5,816 (3,759 - 8,996) |
| 5 | DS2-1 | 27 | 2,663 (1,123 - 6,311) |
| 6 | DS2-1 SK | 28 | 3,299 (2,239 - 4,861) |
| 7 | DS2-2 | 29 | 10,566 (7,794 - 14,324) |
| 8 | DS2-2 SK | 30 | 8,207 (4,200 - 16,038) |
| 9 | DS2-3 | 31 | 9,669 (5,641 - 16,571) |
| 10 | DS2-3 SK | 32 | 7,883 (4,462 - 13,926) |
| 11 | DS2-4 | 33 | 10,010 (7,340 - 13,650) |
| 12 | DS2-4 SK | 34 | 9,805 (5,520 - 17,417) |

### 5. Discussion and Conclusion

This study focused on developing and evaluating prefusion RSV F proteins, particularly the DS-Cav1 and DS2 variants, which were stabilized by the introduction of the SK mutation; these variants were tested using subunit and mRNA vaccine platforms. The SK mutation was designed to improve the structural stability and immunogenicity of these RSV trimeric fusion proteins.

The SK mutation significantly increased the stability of the protein by increasing hydrophobic interaction and decreasing negative charge repulsion between subunits. The *in silico* analysis results showed that the DS-Cav1 SK prefusion F protein had a significantly lower potential energy, indicating increased stability. This improved stability was experimentally confirmed by the DS-Cav1 SK variant showing a significantly higher melting temperature compared to DS-Cav1 in *in vitro* thermal stability testing.

As a result of immunogenicity evaluation in an animal model, the DS-Cav1 SK protein significantly increased neutralizing antibody titers compared to DS-Cav1 in both A2 and B18537 virus strains. This suggests that the SK mutation not only structurally stabilizes the protein but also improves its immunogenic potential. In addition, in this study, the neutralizing antibody response and cross-reactivity of various antigen combinations were evaluated.

The A2 DS2-4 antigen showed a high neutralizing antibody titer against the RSV A2 virus strain but showed only intermediate cross-reactivity against the B18537 virus strain.

On the other hand, the B18357 DS2-4 antigen exhibited strong protection against the B18537 virus strain but showed limited cross-reactivity against the A2 virus strain. When these antigens were combined, the overall cross-reactivity was improved.

In addition, as a result of evaluation of mRNA vaccine candidates, it was confirmed that all tested mRNA vaccines elicited a strong IgG response against the RSV F protein. In particular, the DS2 variant mRNA vaccines had significantly high neutralizing antibody levels, which was consistent with the results of the subunit vaccine experiments.

The introduction of the SK mutation was effective in strengthening the neutralizing antibody response in the DS2 variants and was particularly effective in the DS-Cav1 Group.

In conclusion, introduction of the SK mutation into DS-Cav1 and DS2 variants improves structural stability and immunogenicity.

This study shows that while combining various antigenic viral strains may improve cross-reactivity, the optimal dosage and combination are essential to achieve balanced and robust protection against multiple RSV viral strains. These findings provide a strong foundation for more effective RSV vaccine development and provide valuable insights for future research and development efforts.

From the above description, those of ordinary skill in the art to which the present invention pertains may understand that the present invention may be implemented in other specific forms without changing its technical spirit or essential features. Therefore, it should be understood that the embodiments described above are illustrative in all respects and not restrictive. The scope of the present invention should be interpreted such that all changes or modified forms derived from the meaning and scope of the following patent claims and their equivalent concepts, rather than the detailed description above, are contained in the scope of the present invention.

## Claims

1. A recombinant RSV (Respiratory syncytial virus) F protein, comprising an F2 polypeptide of positions 26-109 and an F1 polypeptide of positions 137-513, based on the sequence of a wild-type RSV F protein precursor polypeptide of SEQ ID NO: 1, and
comprising S155C, S290C, S190F, V207L, D486L, E487L, and F488W amino acid substitutions.

2. The recombinant RSV F protein according to claim 1, comprising an F2 polypeptide of positions 26-103 and an F1 polypeptide of positions 145-513, based on the sequence of the wild-type RSV F protein precursor polypeptide of SEQ ID NO: 1,
wherein a Glycine(Gly)-Serine(Ser) peptide linker is introduced between the amino acid residues at positions 103 and 145, and comprising S155C, S290C, S190F, V207L, L373R, D486L, E487L, and F488W amino acid substitutions.

3. The recombinant RSV F protein according to claim 2, further comprising the following amino acid substitutions:
(a) A149C and Y458C;
(b) N183GC and N428C; or
(c) A149C, Y458C, S46G, E92D, S215P, and K465Q.

4. The recombinant RSV F protein according to claim 1, wherein the recombinant RSV F protein has a trimerization domain linked to the C-terminus directly or via a linker.

5. The recombinant RSV F protein according to claim 4, wherein the trimerization domain is a foldon domain.

6. The recombinant RSV F protein according to claim 1, wherein the recombinant RSV F protein is derived from an F protein of RSV subtype A, an F protein of RSV subtype B, or a bovine RSV F protein.

7. An isolated RNA molecule encoding the recombinant RSV F protein of any one of claims 1 to 6.

8. The nucleic acid according to claim 7, wherein the RNA molecule encodes a precursor polypeptide of a recombinant RSV F protein comprising, in order from the N-terminus to the C-terminus, a signal peptide, an F2 polypeptide, a p27 polypeptide, an extracellular domain of the F1 polypeptide, and a trimerization domain.

9. A vector comprising the RNA molecule of claim 7.

10. An isolated host cell comprising the RNA molecule of claim 7.

11. A method for producing a recombinant RSV F protein, the method comprising:
introducing the RNA molecule of claim 7 into an isolated host cell; and
obtaining the recombinant RSV F protein by culturing the host cell.

12. An immunogenic composition comprising the recombinant RSV F protein of any one of claims 1 to 6, or
the RNA molecule encoding the recombinant RSV F protein.

13. The immunogenic composition according to claim 12, wherein the RNA molecule encodes a recombinant precursor polypeptide of the RSV F protein comprising, in order from the N-terminus to the C-terminus, a signal peptide, an F2 polypeptide, a p27 polypeptide, an extracellular domain of the F1 polypeptide, and a trimerization domain.
